# EUROPEAN PATENT SPECIFICATION

(11) **EP 2 753 425 B1**
(45) Date of publication and mention of the grant of the patent: **18.11.2020**
(21) Application number: 12754035.9
(22) Date of filing: 07.09.2012
(51) Int. Cl.: B01J 41/04, B01J 41/12, B01J 47/12, A61M 1/36, B01J 20/28, B01J 20/32, B01D 69/08, B01D 67/00

(54) **USE OF MODIFIED HOLLOW FIBER MATERIALS FOR REMOVING EXOTOXINS PRODUCED BY ESCHERICHIA COLI FROM BLOOD AND PLASMA**
VERWENDUNG VON MODIFIZIERTEN HOHLFASERMATERIALIEN ZUR ENTFERNUNG VON ESCHERICHIA COLI-EXOTOXINEN AUS BLUT UND PLASMA
UTILISATION DE MATIÈRES MODIFIÉES À FIBRES CREUSES POUR L'ÉLIMINATION D'EXOTOXINES PRODUITES PAR ESCHERICHIA COLI À PARTIR DU SANG ET DU PLASMA

(30) Priority: 08.09.2011 US 201161532255 P; 08.09.2011 EP 11180558
(43) Date of publication of application: 16.07.2014
(73) Proprietor: SAFE bt, Inc., Bethlehem, PA 18018-3524 (US)
(72) Inventor: SEIDEL, Dietrich, 82340 Feldafing (DE); JAEGER, Beate Roxane, 42489 Wülfrath (DE)
(74) Representative: Weickmann & Weickmann PartmbB
(86) International application number: PCT/EP2012/067559
(87) International publication number: WO 2013/034724

(56) References cited:
- EP-A1- 1 602 387
- WO-A1-99/15186
- WO-A1-2011/015197
- JP-A- 2008 255 348
- JP-A- 2009 287 011
- US-A- 5 955 293
- MIYAGAWA A ET AL: "Development of dialyzer with immobilized glycoconjugate polymers for removal of Shiga-toxin", BIOMATERIALS, ELSEVIER SCIENCE PUBLISHERS BV., BARKING, GB, vol. 27, no. 17, 1 June 2006 (2006-06-01), pages 3304-3311, XP027951004, ISSN: 0142-9612 [retrieved on 2006-06-01]

## Description

The present invention relates to chemically modified hollow fiber materials for extracorporeally removing exotoxins produced by pathogenic Escherichia coli from protein containing liquids, particularly from blood or plasma, as well as the use in treating patients suffering from diseases which, for example, are caused by enterohemorrhagic Escherichia coli bacteria (EHEC infections) or other pathogenic Escherichia coli like enterotoxigenic Escherichia coli (ETEC) or enteroaggregative Escherichia coli (EAEC).

Shiga toxin (also known as Shiga-like or Vero toxin) producing Escherichia coli (STEC bacteria) were already described in the year 1977 (Konowalchuk et al., Infect Immun 1977; 18:775-779), but it was not until 1983 that it was associated with the hemolytic uremic syndrome (HUS) (Karmali et al., Lancet 1983; 2:619-20). Since then, especially in case of children, the infection with STEC has demonstrated to be the primary cause of acute kidney failure. It is still today acknowledged that HUS, once it has developed, remains very difficult to treat (Thorpe CM, Food Safety CID. 2004; 38:1298-1303). Thus, efforts are mostly focused on the protection from infection by these pathogenic bacteria.

Since STEC are non-invasive bacteria, a transfer of the Shiga toxins from the intestines into the bloodstream seems to be a precondition for the development of the disease. Additionally, lipopolysaccharides are considered as additional factor in the pathogenesis of HUS (Siegler et. al., Am J Nephrol 2001; 21:420-425 ; Safdar et al., JAMA 2002; 288:996-1001; Dundas et al., Clin Infect Dis 2001; 33:923-931). An estimated 5 - 10% of patients affected with STEC infections suffer from the life-threatening disease HUS.

In Germany and in some bordering countries, an EHEC infection wave occurred without prior warning early in 2011. Despite coordinated efforts by experts, health organisations (including Robert-Koch-Institute), specialised clinics and physicians, a great helplessness became apparent in the treatment of this infection wave, particularly the HUS syndrome. It was the previously largely unknown and aggressive EHEC bacterium O104:H2 which induced a hemolytic uremic syndrome (HUS) in 845 out of 3154 patients infected with EHEC (information Robert-Koch-Institute) and thus led to a situation which was difficult to manage, both, from the clinical and health political point of view. Even an international exchange of experience among experts revealed that so far there are no scientifically based therapeutic measures to counteract this severe disease demonstrably. As a consequence of the helplessness during the infection wave in May, the expenditures were exorbitantly high and have strained the budgets of hospitals as well as general healthcare costs, particularly in the agglomeration areas concerned. Currently there is risk that this situation may repeat itself at any time and any place. This situation, which is unacceptable to modern medical science, makes it necessary to put all scientific efforts into the field of epidemiology, prevention and above all in the treatment of pathogenic Escherichia coli infections, especially EHEC infections.

Stx1-bacteria (Stx1 = Shiga toxin 1) represent a heterogeneous group of organisms having the production of Shiga toxins in common. Hence, in HUS patients, various STEC serotypes were detected and described. Strikingly aggressive is the variant O104:H4 which has recently led to the described infection wave in parts of Central Europe. Today, the diagnosis of the various STEC variants can be performed reliably and relatively fast using molecular genetics methods (PCR). A serological difference is made between two Shiga toxin families (Stx1 and Stx2) wherein toxin variants with diverse degrees of severity can be found. The EHEC strains O154, O26, O123, O11 and O145 are among the common human pathogenic variants and O157:H7 was the very first strain to be associated with gastrointestinal illness. The Shiga toxin consists of one single enzymatic A subunit and five membrane-binding B subunits (AB5-protein). While the B subunits are responsible for the specific binding to their ligand the A subunit is translocated into the target cell what upon activation of the enzyme results in the killing of the cell by protein synthesis inhibition. For the Shiga toxin the receptor on the cell surface is globotriaosylceramide (Gb3) which is present on most eukaryotic cells, in particular enterocytes, epithelial cells and renal cortical cells. The B subunit of the Shiga toxin binds to the terminal trisaccharide of the Gb3 receptor. Shiga toxins released from the bacteria reach the bloodstream only after having damaged and penetrated the intestinal epithelium where by adhering to leukocytes they are transported preferably to endothelial cells of the brain and the kidney (Thorpe CM, Food Safety CID. 2004; 38:1298-1303). These cells possess specific STEC receptors which explains a certain specificity and complication as regards the organs. In the initial state of the disease, in general, the main symptoms are aqueous diarrhea, sometimes followed by bloody diarrhea (observed in 25% of the patients in Germany) and the subsequent occurring HUS with the characteristics of hematological change, kidney failure, epileptic seizures with cerebral edema.

Mortality rate goes up to 10% of the patients. The pathogenic STEC bacteria are mostly transferred via the food chain of humans (meat, vegetables, cucumbers, sprouts, salmon). Also, a transfer from human to human is very likely. In the treatment of this specific bacterial infection, an antibiosis is contra indicated, since this leads to an increase of Shiga toxin concentration in the blood of the patient (Safdar et al., JAMA 2002; 288:996-1001; Dundas et al., Clin Infect Dis 2001; 33:923-931). At present, the emphasis of the therapeutical efforts is placed on hygienic measures in order to avoid the appearance of bacteria in the food chain of humans.

All attempts to reduce the toxin load in the intestines and more importantly in the blood of the infected patients have not yet shown any provable clinical results (Thorpe et al., Food Safety CID. 2004; 38:1298-1303). Medicinal approaches have not gotten beyond animal testing (pig, mouse). A first clinical trial was stopped due to ineffectiveness (Trachtman et al., JAMA 2003; 290:1337-1344).

Thus, to this day, no medicaments, substances or measures in terms of medical technology are available that have shown a target-oriented treatment and improvement of the pathogenesis of the Shiga toxin disease in humans. This applies also, for instance, for:
a) synthetic toxin binders for oral administration (Trachtman et al., JAMA 2003; 290:1337-1344; Watanabe et al., J Infect Dis 2004; 189:360-368)
b) probiotic bacteria which shall compete with the Shiga toxin via specific cell surface proteins (Gb3-receptors) (Pinyon et al., J Infect Dis 2004; 189:1547-1555) or
c) tests with monoclonal antibodies directed against Shiga-toxins (Mukherjee et al., Infect Immun 2002; 70:5896-5899; Sheoran et al., Infect Immun 2003; 71:3125- 3130).

All known therapeutic measures were based on the attempt to benefit from a toxin binding in the intestinal lumen. An appropriate approach which should and will be pursued further in the future.

Furthermore, also an approach regarding the engineering of recombinant bacteria competing for the binding receptors of *Stx1* and *Stx2* producing strains is potentially interesting (Pinyon et al., J Infect Dis 2004; 189:1547-1555). Also the use of monoclonal antibodies or antibody mixtures directed against Shiga toxins seems to be attractive and is theoretically interesting since it follows an approach of many other diseases. However, all treatment measures which are theoretically possible regarding pathogenic EHEC infections have so far not been satisfying from a clinical point of view.

A further approach is the development of a dialyzer with immobilized glycoconjugate polymers having oligosaccharides and amino groups to function as Shiga-toxin adsorbents (Miyagawa A, Watanabe M, Igai K, et al. Development of dialyzer with immobilized glycoconjugate polymers for removal of Shiga-toxin. Biomaterials. 2006;27(17):3304-3311).

In the summary of the current state of knowledge regarding the treatment of pathogenic Escherichia coli infections, especially EHEC infections and, moreover, of HUS, it is apparent that the research-based and clinical medicine still lacks efficient measures against this threatening infectious disease. On the clinical level, the attempt to cope with this problem by means of plasma exchange treatment could not convince either. This is a non-selective and not very efficient procedure. Besides the intended elimination of Shiga toxins, protective and anti-inflammatory mediators are simultaneously extracted from the patient. Such a plasma exchange needs an exchange volume of approximately 12 liters of plasma per patient which requires a large number of donors and which can be accompanied by a higher risk of additional infections and allergic reactions. Furthermore, a plasma exchange treatment involves exorbitant treatment costs.

Thus, the present invention is based on the object to provide a treatment method and corresponding materials as well as an appropriate device by means of which the disadvantages of the recent therapeutic measures can be avoided and which generally allow an effective treatment of patients infected with pathogenic Escherichia coli, especially EHEC. This problem is solved by means of a method and corresponding materials or a device that had originally been developed for removing bacterial endotoxins (lipopolysaccharides, LPS) and lipoteichonic acids (LTA) from the blood or plasma of patients in an extracorporeal perfusion system as described in EP 1 602 387.

The present invention relates to a substance for use in the treatment of the hemolytic uremic syndrome (HUS) caused by exotoxins, especially Shiga toxins, produced by Escherichia coli, and if applicable HUS sequelae, by binding and removing toxins from blood or plasma of a patient, the substance containing modified hollow fiber materials selected from the group consisting of polyamide, polysulfone, polyether, polyethylene, polypropylene, polyester and derivatives and/or mixtures thereof, wherein said hollow fiber materials are modified to contain tentacles comprising anion exchange groups, wherein the anion exchange groups comprise at least one of the group consisting of synthetic polycationic chains and semisynthetic polycationic chains and natural polycationic chains, wherein said synthetic, semisynthetic and natural polycationic chains are linear or branched and wherein the anion exchange groups consist of polycationic chains containing tertiary amino or quaternary ammonium groups.

It was surprisingly found that by this substance and a device containing this substance, Shiga toxins can efficiently be removed from the blood or plasma by means of adsorption although the bacterial toxins LPS and LTA generally differ in their chemical structure and biochemical function from the Shiga toxins. LPS and LTA are oligo-/polysaccharides esterified with fatty acids and they are part of the membrane of gram-negative and gram-positive bacteria.
Shiga toxins, however, are enzymatically active, intracellular proteins (exotoxins) of gram-negative bacteria.

Patients infected with pathogenic Escherichia coli may in case of an enterotoxigenic Escherichia coli infection suffer from different kinds of diarrhea which can be watery, mild, brief, self-limiting, weanling diarrhea among children, diarrhea resulting in severe purging or traveler's diarrhea; a watery, mucoid, bloody and secretory diarrheal illness with low grade fever and little to no vomiting has been described for patients infected with enteroaggregative Escherichia coli. After 1-2 days post infection with enterohemorrhagic Escherichia coli the diarrhea becomes bloody accompanied with abdominal pain which may last between 4 to 10 days and in some cases (5 - 10 %) the disease may progress to HUS.

As used herein the term "exotoxin" refers to any exotoxin produced by any pathogenic Escherichia coli strain, preferably ETEC, EAEC, and most preferably EHEC.

It is preferred to provide the substance according to the invention in a device which is arranged in the form of a filter module and most preferably is useful for application in a plasma perfusion/adsorption apheresis system. For a device to be used for adsorption apheresis, among other things the following conditions must be met:
1. Elimination of exotoxins should be as selective and efficient as possible.
2. The binding capacity of the adsorbents used should satisfy optimum practical requirements.
3. It must be possible to sterilize the adsorbents with heat or gamma radiation, without loss or alteration of their properties.
4. The adsorbents should permit a sufficiently high flow rate of up to 200 ml/min.
5. The method of elimination must display the medically necessary biocompatibility and hemocompatibility and must not impair any physiological control systems and protective mechanisms, for example the immune, complement or coagulation system.

A person skilled in the art will be able to find suitable hollow fiber adsorbent materials according to this profile of requirements. In principle, hollow fiber materials can be used that are made from polyamide, polysulfone, polyether, polyethylene, polypropylene, polyester or derivatives and/or mixtures of these polymers. In an especially preferred embodiment of the invention the hollow fibers consist of polyamid, preferably nylon.

Suitable hollow fiber materials according to the invention are based on affinity membranes, as described for example in U.S. Pat. Nos. 5,053,133, 5,766,908, and in WO96/22316. These membrane base materials can be modified by known methods and in particular by graft polymerization, in this connection see for example WO96/22316, in which an appropriate method is described. Other derivatization methods for appropriate polymer materials, suitable for the production of hollow fiber adsorbent materials, i.e. the substance according to the invention, are also known to a person skilled in the art and can be used within the scope of the present invention.

The substances according to the invention are preferably arranged in a device in such a way that there is effective flow of blood or plasma through them, ensuring maximum wetting of the hollow fiber membrane so that optimum adsorption of the exotoxin onto the hollow fiber adsorbent materials can take place. Preferred arrangements of the hollow fiber materials in a device according to the invention are disclosed in WO98/57733, WO98/33581, WO98/19777 or WO98/28064, and an especially preferred arrangement is moreover described in EP 1 002 566.

As well as the hollow fiber materials, a device according to the invention can have additional materials, e.g. flat membranes, between the hollow fibers, which can often give a further increase in adsorption and separation performance. Corresponding adsorber arrangements are described in EP 0 956 147.

Both the materials disclosed in the aforesaid documents and the methods of modification and ultimate arrangements in adsorbers can be employed in the device according to the invention.

To make the removal of exotoxins from blood or plasma particularly efficient, the device employs hollow fibers that are modified chemically in such a way that the exotoxin is bound particularly well to the hollow fiber material and is therefore removed from the blood or plasma. Preferably, chemical modification of the hollow fiber material is carried out, and graft polymerization (see above) has proved especially favorable for the said modification, compounds being grafted onto the hollow fiber material that display good binding capacity for exotoxins. Grafting-on of anion exchanger groups has also proved especially advantageous. These anion exchanger groups are especially advantageous when in the form of longer chains with a large number of cationic groups, called tentacles. These tentacle-like extensions on the base material are able to bind exotoxin molecules, producing a further increase in efficiency of the hollow fiber material. This modification of the hollow fiber material by means of tentacles preferably employs synthetic and/or semisynthetic and/or natural polycationic chains, and the said chains can be in linear or branched form. Modification of the hollow fiber materials according to the invention with cationic or polycationic chains that have tertiary amino and/or quaternary ammonium groups is especially preferred.

A further possibility to immobilize the anion exchanger groups on the hollow fiber material are standard techniques used for chemical immobilization which are known to an person skilled in the art.

Preferred anion exchanger groups on the hollow fiber materials include di- or trialkylaminoalkyl, di- or trialkylaminoaryl-, di- or triarylaminoalkyl, di- or triarylaminoaryl, di- or trialkylammoniumalkyl- di- or triarylammoniumalkyl, di-or triarylammoniumaryl- and di- or trialkylammoniumaryl residues. Furthermore, polymers from amino acids that are positively charged or contain tertiary amino or quaternary ammonium groups, such as polylysine, polyarginine or polyhistidine or copolymers or derivatives thereof are suitable as anion exchanger materials within the scope of the invention, as well as polyethylene-imine.

In quite especially preferred embodiments of the invention, the device contains a polyamide hollow fiber material modified with diethylaminoalkyl or diethylaminoaryl residues, in particular diethylaminoethyl polyamide. Furthermore, it is preferred to arrange the device in such a way that it can be used as a replaceable filter cartridge for an existing perfusion system. This can be inserted easily into the perfusion system and can have a small volume, on account of the high binding capacity and specificity of the hollow fiber material used, so that a greatly reduced dead volume can be achieved in comparison with systems described hitherto. Surprisingly, a device according to the invention can be made as a cartridge with dimensions of for example 12 cm long and 5 cm in diameter, which can be used extremely effectively in an extracorporeal perfusion system. The cartridge in this example has a dead volume of only approx. 115 ml. Therefore it is especially preferred, within the scope of the present invention, to dimension the cartridges so that the dead volume is <150 ml and preferably 80 to 130 ml. As it could be shown that the said filter cartridges can remove exotoxins, especially Shiga toxins effectively and selectively, merely this small dead volume provides surprising and considerable advantages relative to the state of the art in addition to the possibility newly discovered according to the invention, of removing exotoxins, especially Shiga toxins and LPS simultaneously in one implementation. After the end of treatment and cleaning of the apparatus or even for carrying out an extended (continuous) blood or plasma perfusion, a new cartridge can simply be inserted.

Within the scope of the present invention, it was found that the relevant hollow fiber materials eliminate exotoxins, especially Shiga toxins at physiological pH by adsorption from whole blood and/or blood plasma, at high selectivity and capacity.

It was found that even at physiological pH, only a small, compositionally safe amount of blood and plasma proteins are adsorbed. Fibrinogen, in particular, is only removed from the patient's blood to a quite small extent (<2%) within the scope of the present invention, so that there is hardly any impairment of the natural coagulation cascade when using the device according to the invention.

Further described is the use of the hollow fiber materials described, and contained in the device according to the invention, for making a means for eliminating exotoxin from body fluids, in particular from blood or plasma.

The hollow fiber materials used according to the invention, which are preferably selected from the group comprising polyamides, polysulfones, polyethers, polyethylene, polypropylene or polyesters and derivatives and/or mixtures of these materials, permit effective elimination of exotoxins, especially Shiga toxins from a patient's blood circulation and can therefore be used advantageously in an adsorption apheresis apparatus. As a rule, in such apparatus there will firstly be separation of whole blood into plasma and corpuscular blood components, then the plasma is directed over the adsorbent material and after that the corpuscular blood components are returned to it.

The hollow fiber materials used as adsorbents according to the invention are, in a preferred embodiment, modified chemically in such a way that optimum exotoxin adsorption can take place thereon. Modification of the hollow fibers by graft polymerization is especially preferred, and the grafting-on of anion exchanger groups, especially in the form of so-called tentacles, i.e. chain-like, branched molecules with as many anion exchanger groups as possible, is preferred.

An especially preferred hollow fiber material is a DEAE-modified polyamide.

As already mentioned above, with respect to the device according to the invention, it is advantageous to arrange the means in the form of a filter, if possible in the form of a disposable cartridge, ensuring easy and safe use.

Also described is the use of the device according to the invention or of the means produced according to the invention for removing exotoxins from body fluids, especially blood or plasma.

Especially advantageously, the device with the substance according to the invention is used in an extracorporeal perfusion system, which provides particularly effective removal of bacterial exotoxins from the patient's blood and leads to exceptionally successful treatment of patients. During application moreover, exotoxins are removed very selectively, whereas endogenous proteins are only removed to a very small extent, if at all, or said removal only applies to proteins that are easily replaceable and whose removal is not notably stressful for the already much debilitated patient. Treatment of the patients can be curative and/or prophylactic. Thus, a treatment can be used with patients suffering from an infection by pathogenic Escherichia coli in order to cure the intestinal disease together with sequelae associated with the infection like, for example, HUS in case of an EHEC infection. The treatment can be considered as prophylactic as it reduces the risk of a progression to HUS.

Further described is the use the adsorbent materials described above for the isolation, enrichment, and detection of exotoxins, especially Shiga toxins. The said isolation, enrichment, and detection can take place from any fluids, preferably from blood or plasma, and this can be done for any purposes, though in particular for analysis and/or diagnosis.

The following examples should further illustrate the present invention.

### Examples

### Example 1: Elimination of Shigatoxin by selective adsorption under in vitro conditions

### A) Preparation of Shigatoxin

For preparation and enrichment of Shigatoxin E. coli stem EHEC 0104 was incubated overnight in a medium containing: NaCl (10 g/L), Triptone (10 g/L, Becton Dickinson) and yeast-extract (5 g/L, Becton Dickinson). After centrifugation (200 g, 30 min) of the suspension and sedimentation of the E.coli bacteria a supernatant was obtained which has been highly enriched with Shigatoxin 2 (Stx2). The concentration of Stx2 corresponded to an "optical density" (OD 450/620) of 4.06. This value has been found by applying a dedicated ELISA-test kit (Premier EHEC, Meridian Bioscience).

### B) Perfusion of Shigatoxin solution through the S.A.F.E. bt adsorber (B. Braun Melsungen AG)

An aliquot of the supernatant obtained following the procedure described in A) and which contains Stx2 was diluted 1:2. This solution was perfused through the S.A.F.E. bt adsorber respectively (4 times) by applying a negative pressure to the adsorber cartridge. The data obtained for the corresponding perfusates are listed in Table 1.

**Table 1: Eliminationrate for Shigatoxin**

| | OD 450/620 | Eliminationrate [%] |
|---|---|---|
| Stx2-Solution (untreated) | 2.033 | --- |
| 1. Perfusate | 0.286 | 86 |
| 2. Perfusate | 0.269 | 87 |
| 3. Perfusate | 0.230 | 89 |
| 4. Perfusate | 0.190 | 91 |

### Example 2: Prophylactic treatment of EHEC

This case is about a 17-year old boy, who suffered from bloody diarrhea during three days and whose faeces were positive for EHEC. A hemolytic-uremic syndrome (HUS) was not confirmed at that time. Four days after he had developed diarrhea for the first time (on June 21, 2011), the patient was treated with the SAFE-system. The duration of treatment was two hours. The therapy was carried out without any complications and it was well tolerated by the patient. After the treatment and during the following days, no diarrhea occurred. The shiga toxin was no longer detectable in the faeces and the patient could leave the hospital after a few days without showing any symptoms. During the follow-up examination on July 07, 2011, no EHEC-bacteria could be detected and the patient's results were normal.

### Example 3: Curative treatment of the EHEC/HUS-syndrome

This case is about a 74-year old women from Hannover. The patient was in good health when she suddenly developed diarrhea (non-bloody) during four days. On June 16, 2011, she was moved from the hospital in Lehrte to the Oststadt-Heidehaus clinic in Hannover since she had developed acute renal failure with a suspected HUS syndrome.

When she was admitted to hospital on June 16, 2011, the patient was awake and responsive. However, according to the admitting physician's description of the patient's neurological status, she was "anxious and forgetful". When she was admitted to hospital, the laboratory parameters showed a creatine concentration of 7.2 mg/dl, a urea concentration of 220 mg/dl, a blood platelet concentration of 115 T/ml and a lactic dehydrogenase of 1460 U/L.

Between June 16, 2011, and June 22, 2011, the patient received acute dialysis in the intensive care unit and due to the EHEC-HUS syndrome, which had meanwhile been confirmed, she was treated with plasmapheresis, eculizimab and antibiotics four times a day. Despite the therapy, the patient developed an increasing neurological involvement: at first aphasia, then generalized seizures as well as increasing obtundation. During CCT, no intracerebral haemorrhage was detected.

Due to the protracted obtundation, it was decided - as ultima ratio - to carry out a SAFE-treatment. When the treating doctor first met the patient on June 22, 2011, she was still completely anuric. She was not responsive, it was only possible to wake her up by means of pain stimuli, however, she was aphasic. The neurological symptoms included abducens paralysis on both sides, undergone generalized seizures and a hemiparesis on the left side.

The first treatment was carried out on Wednesday, June 22, 2011, in the afternoon. After having treated 12l of plasma (approx. 2-3 hours after the therapy had been started), the patient was again responsive and, for the first time, she was able to answer questions regarding her orientation in a correct and comprehensible manner. Furthermore, she was also able to eat and drink on her own for the first time.

On June 23, 2011, according to the treatment regimen, she was again treated with antibodies.

Between June 24, 2011, and June 26, 2011 (i.e., from Friday until Sunday night) she was again treated with the SAFE-system; this time, continuously over three days (> 181 of plasma volume with a system without any complications). Since the first SAFE-treatment, no further neurological complications could be observed and the patient's condition visibly stabilized.

The following Monday, on June 27, 2011, the patient was already moved to the normal care unit since also the renal function continued to recover. Until July 08, 2011, the patient's symptoms had continuously receded to a great extent. The last creatine concentration was 2.7 mg/dl, measured on July 08, 2011.

## Claims

1. Substance for use in the treatment of the hemolytic uremic syndrome (HUS) caused by exotoxins, especially Shiga toxins, produced by Escherichia coli, and if applicable HUS sequelae, by binding and removing toxins from blood or plasma of a patient, the substance containing modified hollow fiber materials selected from the group consisting of polyamide, polysulfone, polyether, polyethylene, polypropylene, polyester and derivatives and/or mixtures thereof, wherein said hollow fiber materials are modified to contain tentacles comprising anion exchange groups, wherein the anion exchange groups comprise at least one of the group consisting of synthetic polycationic chains and semisynthetic polycationic chains and natural polycationic chains, wherein said synthetic, semisynthetic and natural polycationic chains are linear or branched and wherein the anion exchange groups consist of polycationic chains containing tertiary amino or quaternary ammonium groups.

2. Substance for use according to claim 1,
wherein hollow fibers are used that were modified by graft polymerization and/or chemical immobilisation.

3. Substance for use according to any one of the preceding claims,
wherein anion exchanger groups are selected from the group consisting of dialkylaminoalkyl, dialkylaminoaryl, diarylaminoalkyl, diarylaminoaryl, trialkylammoniumalkyl, triarylammoniumalkyl, triarylammoniumaryl, trialkylammoniumaryl residues, polymers from amino acids that are positively charged or contain tertiary amino or quaternary ammonium groups as polylysine, polyarginine or polyhistidine or mixtures thereof or polyethylene-imine.

4. Substance for use according to any one of the preceding claims,
wherein the hollow fiber material is a polyamide modified with diethylaminoethyl groups.

5. Substance for use according to any one of the preceding claims,
wherein the hollow fiber material is nylon.

6. Substance for use according to any one of the preceding claims,
wherein the substance is provided in a device which is arranged in the form of a filter module.

7. Substance for use according to claim 6,
wherein the filter module has a dead volume of <150 ml, preferably 80-130ml.

8. Substance for use according to claim 6 or 7,
wherein the device permits a flow rate of up to 200 ml/minute.

9. Substance for use according to any one of the preceding claims,
wherein said device is designed for application in a plasma perfusion system.

10. Substance for use according to any one of the preceding claims,
wherein the treatment can be prophylactic and/or curative.

11. Substance for use according to any one of the preceding claims,
wherein it binds exotoxins produced by enterohemorrhagic Escherichia coli, enterotoxigenic Escherichia coli or enteroaggregative Escherichia coli.

12. Substance for use according to any one of the preceding claims,
wherein it binds toxins produced by enterohemorrhagic Escherichia coli.

13. Substance for use according to any one of the preceding claims,
wherein the toxins belong to the family of the Shiga toxins.

14. Substance for use according to any one of the preceding claims,
wherein it simultaneously binds exotoxins, preferably Shiga toxins, and lipopolysaccharides (LPS).

## Patentansprüche

1. Substanz zur Verwendung bei der Behandlung des hämolytisch-urämischen Syndroms (HUS), das durch Exotoxine, insbesondere Shiga-Toxine, hergestellt durch Escherichia coli, verursacht wird und falls zutreffend HUS Folgeerscheinungen, durch Binden und Entfernen von Toxinen aus dem Blut oder Plasma eines Patienten, wobei die Substanz modifizierte Hohlfasermaterialien enthält, ausgewählt aus der Gruppe bestehend aus Polyamid, Polysulfon, Polyether, Polyethylen, Polypropylen, Polyester und Derivaten und/oder Mischungen davon, wobei die Hohlfasermaterialien modifiziert sind, um Tentakeln zu enthalten, die Anionenaustauschgruppen umfassen, wobei die Anionenaustauschgruppen mindestens eine der Gruppe umfasst, bestehend aus synthetischen polykationischen Ketten und halbsynthetischen polykationischen Ketten und natürlichen polykationischen Ketten, wobei die synthetischen, halbsynthetischen und natürlichen polykationischen Ketten linear oder verzweigt sind und wobei die Anionenaustauschgruppen aus polykationischen Ketten bestehen, die tertiäre Amino- oder quaternäre Ammoniumgruppen enthalten.

2. Substanz zur Verwendung nach Anspruch 1, wobei Hohlfasern verwendet werden, die durch Pfropfpolymerisation und/oder chemische Immobilisierung modifiziert wurden.

3. Substanz zur Verwendung nach einem der vorherigen Ansprüche, wobei Anionenaustauschergruppen ausgewählt sind aus der Gruppe bestehend aus Dialkylaminoalkyl-, Dialkylaminoaryl-, Diarylaminoalkyl-, Diarylaminoaryl-, Trialkylammoniumalkyl-, Triarylammoniumalkyl-, Triarylammoniumaryl-, Trialkylammoniumarylresten, Polymeren aus Aminosäuren, die positiv geladen sind oder tertiäre Amino- oder quaternäre Ammoniumgruppen enthalten, wie Polylysin, Polyarginin oder Polyhistidin oder Mischungen davon oder Polyethylenimin.

4. Substanz zur Verwendung nach einem der vorherigen Ansprüche,
wobei das Hohlfasermaterial ein mit Diethylaminoethylgruppen modifiziertes Polyamid ist.

5. Substanz zur Verwendung nach einem der vorherigen Ansprüche,
wobei das Hohlfasermaterial Nylon ist.

6. Substanz zur Verwendung nach einem der vorherigen Ansprüche,
wobei die Substanz in einer Vorrichtung bereitgestellt wird, die in Form eines Filtermoduls angeordnet ist.

7. Substanz zur Verwendung nach Anspruch 6,
wobei das Filtermodul ein Totvolumen von <150 ml, vorzugsweise 80- 130 ml, aufweist.

8. Substanz zur Verwendung nach Anspruch 6 oder 7,
wobei die Vorrichtung eine Fließrate von bis zu 200 ml/Minute ermöglicht.

9. Substanz zur Verwendung nach einem der vorherigen Ansprüche,
wobei die Vorrichtung für die Anwendung in einem Plasma-Perfusionssystem entwickelt ist.

10. Substanz zur Verwendung nach einem der vorherigen Ansprüche,
wobei die Behandlung prophylaktisch und/oder kurativ sein kann.

11. Substanz zur Verwendung nach einem der vorherigen Ansprüche,
wobei sie Exotoxine bindet, die von enterohämorrhagischen Escherichia coli, enterotoxigenen Escherichia coli oder enteroaggregativen Escherichia coli hergestellt werden.

12. Substanz zur Verwendung nach einem der vorherigen Ansprüche,
wobei sie Toxine bindet, die von enterohämorrhagischen Escherichia coli hergestellt werden.

13. Substanz zur Verwendung nach einem der vorherigen Ansprüche,
wobei die Toxine zur Familie der Shiga-Toxine gehören.

14. Substanz zur Verwendung nach einem der vorherigen Ansprüche,
wobei sie gleichzeitig Exotoxine, vorzugsweise Shiga-Toxine, und Lipopolysaccharide (LPS) bindet.

## Revendications

1. Substance destinée à être utilisée dans le traitement du syndrome hémolytique et urémique (SHU) causé par des exotoxines, en particulier les shigatoxines, produites par Escherichia coli, et le cas échéant les séquelles du SHU, en liant et retirant les toxines du sang ou du plasma d'un patient, la substance contenant des matériaux en fibres creuses modifiés choisis dans le groupe consistant en polyamide, polysulfone, polyéther, polyéthylène, polypropylène, polyester et leurs dérivés et/ou mélanges, où lesdits matériaux en fibres creuses sont modifiés pour contenir des tentacules comprenant des groupes échangeurs d'anions, où les groupes échangeurs d'anions comprennent au moins l'un du groupe consistant en chaînes polycationiques synthétiques et en chaînes polycationiques semi-synthétiques et en chaînes polycationiques naturelles, où lesdites chaînes polycationiques synthétiques, semi-synthétiques et naturelles sont linéaires ou ramifiées et où les groupes échangeurs d'anions consistent en chaînes polycationiques contenant des groupes amino tertiaires ou ammonium quaternaires.

2. Substance destinée à être utilisée selon la revendication 1, où des fibres creuses qui ont été modifiées par polymérisation par greffage et/ou immobilisation chimique sont utilisées.

3. Substance destinée à être utilisée selon l'une quelconque des revendications précédentes,
où les groupes échangeurs d'anions sont choisis dans le groupe consistant en résidus de dialkylaminoalkyle, dialkylaminoaryle, diarylaminoalkyle, diarylaminoaryle, trialkylammoniumalkyle, triarylammoniumalkyle, triarylammoniumaryle, trialkylammoniumaryle, polymères d'acides aminés qui sont chargés positivement ou contiennent des groupes amino tertiaires ou ammonium quaternaires comme la polylysine, la polyarginine ou la polyhistidine ou leurs mélanges ou la polyéthylène-imine.

4. Substance destinée à être utilisée selon l'une quelconque des revendications précédentes,
où le matériau en fibres creuses est un polyamide modifié avec des groupes diéthylaminoéthyle.

5. Substance destinée à être utilisée selon l'une quelconque des revendications précédentes,
où le matériau en fibres creuses est le nylon.

6. Substance destinée à être utilisée selon l'une quelconque des revendications précédentes,
où la substance est fournie dans un dispositif qui est agencé sous forme d'un module de filtre.

7. Substance destinée à être utilisée selon la revendication 6,
où le module de filtre a un volume mort <150 ml, de préférence de 80-130 ml.

8. Substance destinée à être utilisée selon la revendication 6 ou 7,
où le dispositif permet un débit de jusqu'à 200 ml/minute.

9. Substance destinée à être utilisée selon l'une quelconque des revendications précédentes,
où ledit dispositif est conçu pour une application dans un système de perfusion de plasma.

10. Substance destinée à être utilisée selon l'une quelconque des revendications précédentes,
où le traitement peut être prophylactique et/ou curatif.

11. Substance destinée à être utilisée selon l'une quelconque des revendications précédentes,
où elle se lie aux exotoxines produites par Escherichia coli entérohémorragique, Escherichia coli entérotoxigène ou Escherichia coli entéro-agrégatif.

12. Substance destinée à être utilisée selon l'une quelconque des revendications précédentes,
où elle se lie aux toxines produites par Escherichia coli entérohémorragique.

13. Substance destinée à être utilisée selon l'une quelconque des revendications précédentes,
où les toxines appartiennent à la famille des shigatoxines.

14. Substance destinée à être utilisée selon l'une quelconque des revendications précédentes,
où elle se lie simultanément à des exotoxines, de préférence des shigatoxines, et des lipopolysaccharides (LPS).
